# EUROPEAN PATENT APPLICATION

(11) **EP 4 147 731 A1**
(43) Date of publication of application: **15.03.2023**
(21) Application number: 21800873.8
(22) Date of filing: 06.05.2021
(51) Int. Cl.: A61L 31/14, A61L 31/04, A61L 31/06

(54) **HEMOSTATIC POLYMER MATERIAL KIT**

(30) Priority: 08.05.2020 JP 2020082474
(71) Applicant: The University of Tokyo, Bunkyo-ku, Tokyo 113-8654 (JP); Gellycle Co., Ltd., Tokyo 113-0033 (JP)
(72) Inventor: SAKAI Takamasa, Tokyo 113-8654 (JP); MASUI Kosuke, Tokyo 113-0033 (JP); NARITA Shinichi, Tokyo 113-0033 (JP); KAMATA Hiroyuki, Tokyo 113-0033 (JP)
(74) Representative: Symbiosis IP Limited
(86) International application number: PCT/JP2021/017403
(87) International publication number: WO 2021/225144

(57) **Abstract**

An object of the present invention is to provide a hemostatic method, a vascular occlusion method, a tissue covering method, and a body fluid coagulation method that are safe and efficient using a polymer material that is a non-biologically derived synthetic product.

A solution (pre-gel solution) is prepared that contains, under a specific concentration condition, a hydrophilic polymer capable of forming a hydrogel by intermolecular crosslinking and has a specific pH condition and specific ionic strength, and the solution is gelled in situ in an environment, such as a bleeding site or the interior of a blood vessel, where blood is present.

## Description

### Technical Field

The present invention relates to a polymer material kit for hemostasis, vascular occlusion, tissue covering, or body fluid coagulation, and further relates to a hemostatic method, a vascular occlusion method, a tissue covering method, and a body fluid coagulation method in which the polymer material kit is used.

### Background Art

Conventional methods used for blood coagulation mainly include a method of accelerating a hemostatic reaction by using a pharmacological action of a biologically derived blood coagulation factor represented by a fibrin glue (for example, Patent Literature 1) and a method of physically blocking a blood flow and coagulating the blood by the coagulability of the blood itself.

However, the former method in which a biologically derived material is used has an infection risk because infection control is not easy in an animal as a raw material or in a donor. In fact, problems have occurred such as HIV infection through unheated blood products and iatrogenic Creutzfeldt-Jakob disease. Meanwhile, the latter method in which physical blocking is used is difficult to apply to an affected part having a complicated structure because a sheet-like material is to be used due to the characteristics of the hemostasis principle. Furthermore, both the methods have a common problem that a blood coagulation effect is difficult to obtain for a patient to whom an agent such as an anticoagulant is administered because the final hemostatic state depends on the blood coagulability of the patient.

### Citation List

### Patent Literature

Patent Literature 1: JP 2017-66150 A

### Summary of Invention

### Technical Problem

Therefore, an object of the present invention is to provide a hemostatic method, a vascular occlusion method, a tissue covering method, and a body fluid coagulation method that are safe and efficient using a polymer material that is a non-biologically derived synthetic product.

### Solution to Problem

As a result of intensive studies to solve the above-described problems, the present inventors have found that an excellent blood coagulation effect and an excellent hemostatic effect can be obtained by preparing a solution (pre-gel solution) that contains, under a specific concentration condition, a hydrophilic polymer capable of forming a hydrogel by intermolecular crosslinking and has a specific pH condition and specific ionic strength, followed by forming the solution into a gel in situ in an environment, such as a bleeding site or the interior of a blood vessel, where blood is present, and thus the present invention has been completed.

That is, in one aspect, the present invention relates to a kit for formation of a hydrogel in an environment where blood is present, and specifically provides:
<1> A polymer material kit including a polymer solution A containing a first polymer and a second polymer solution B containing a second polymer, the first polymer and the second polymer being a hydrophilic polymer having a polyalkylene glycol skeleton or a polyvinyl skeleton, wherein a combination of the first polymer and the second polymer is capable of forming a hydrogel when the first polymer and the second polymer are crosslinked with each other, the first polymer and the second polymer have a weight average molecular weight (Mw) in a range of 1 × 10³ to 1 × 10⁵, the polymer solution A and the second polymer solution B have a concentration of the first polymer and a concentration of the second polymer in a range of 10 to 300 g/L, respectively, and a mixed solution obtained by mixing the polymer solution A and the second polymer solution B has a pH of 3 to less than 7 and an ionic strength in a range of 10 to 100 mM;
<2> The polymer material kit according to the item <1>, wherein the first polymer and the second polymer are a two-, three-, or four-armed polyethylene glycol;
<3> The polymer material kit according to the item <1> or <2>, wherein the first polymer has one or more nucleophilic functional groups in a side chain or at a terminal, and the second polymer has one or more electrophilic functional groups in a side chain or at a terminal;
<4> The polymer material kit according to any one of the items <1> to <3>, wherein the one or more nucleophilic functional groups are selected from the group consisting of a thiol group and an amino group, and the one or more electrophilic functional groups are selected from the group consisting of a maleimidyl group, an N-hydroxy-succinimidyl (NHS) group, a sulfosuccinimidyl group, a phthalimidyl group, an imidazoyl group, an acryloyl group, a nitrophenyl group, and -CO₂PhNO₂;
<5> The polymer material kit according to any one of the items <1> to <4>, wherein the polymer solution A and the second polymer solution B have a pH in a range of 3 to less than 7;
<6> The polymer material kit according to any one of the items <1> to <5>, wherein a hydrogel in which the first polymer and the second polymer are crosslinked with each other is formed when the polymer solution A and the second polymer solution B are mixed in an environment where a liquid having a pH of 6.5 to 8.0 is present;
<7> The polymer material kit according to the item <6>, wherein the hydrogel formed using the first polymer and the second polymer has a gelation time in a range of 1 to 30 seconds;
<8> The polymer material kit according to the item <6> or <7>, wherein the hydrogel formed using the first polymer and the second polymer has an equilibrium swelling degree in a range of 0.9 to 3.5;
<9> The polymer material kit according to any one of the items <6> to <8>, wherein the hydrogel formed using the first polymer and the second polymer has a Young's modulus in a range of 0.1 × 10⁴ to 4 × 10⁴ Pa;
<10> The polymer material kit according to any one of the items <1> to <9>, to be used for hemostasis, vascular occlusion, tissue covering, or body fluid coagulation;
<11> A hemostatic agent including the polymer material kit according to any one of the items <1> to <10>;
<12> A vascular occlusion agent including the polymer material kit according to any one of the items <1> to <10>;
<13> A tissue covering agent including the polymer material kit according to any one of the items <1> to <10>; and
<14> A body fluid coagulant including the polymer material kit according to any one of the items <1> to <10>.

In another aspect, the present invention relates to a method for producing a hemostatic agent or the like containing a hydrogel using the kit, and specifically provides:
<15> A method for producing a hemostatic agent, a vascular occlusion agent, a tissue covering agent, or a body fluid coagulant containing a hydrogel, the method including a step of applying a mixed solution of a polymer solution A containing a first polymer and a second polymer solution B containing a second polymer to an environment where a liquid having a pH of 6.5 to 8.0 is present, wherein the first polymer and the second polymer are a hydrophilic polymer having a polyalkylene glycol skeleton or a polyvinyl skeleton, a combination of the first polymer and the second polymer is capable of forming a hydrogel when the first polymer and the second polymer are crosslinked with each other, the first polymer and the second polymer have a weight average molecular weight (Mw) in a range of 1 × 10³ to 1 × 10⁵, the polymer solution A and the second polymer solution B have a concentration of the first polymer and a concentration of the second polymer in a range of 10 to 300 g/L, respectively, and the mixed solution obtained by mixing the polymer solution A and the second polymer solution B has a pH of 3 to less than 7 and an ionic strength in a range of 10 to 100 mM;
<16> The method according to the item <15>, including mixing the polymer solution A and the second polymer solution B in an environment where a liquid having a pH of 6.5 to 8.0 is present;
<17> The method according to the item <15>, including dripping the polymer solution A and the second polymer solution B on a carrier and then bringing the carrier into contact with an environment where a liquid having a pH of 6.5 to 8.0 is present; and
<18> The method according to any one of the items <15> to <17>, wherein the polymer solution A and the second polymer solution B have a pH in a range of 3 to less than 7.

In another aspect, the present invention also relates to a hemostatic method and the like in which the kit is used, and specifically provides:
<19> A hemostatic method including using the polymer material kit according to any one of the items <1> to <10>;
<20> A vascular occlusion method including using the polymer material kit according to any one of the items <1> to <10>;
<21> A tissue covering method including using the polymer material kit according to any one of the items <1> to <10>;
<22> A body fluid coagulation method including using the polymer material kit according to any one of the items <1> to <10>;
<23> A medical device including the polymer material kit according to any one of the items <1> to <10>; and
<24> The medical device according to the item <23>, wherein at least one of the polymer solution A or the second polymer solution B is stored in a sprayer.

### Advantageous Effects of Invention

According to the polymer material kit of the present invention, by applying two polymer solutions to an environment where blood is present such as a bleeding site or the interior of a blood vessel, a change in pH is caused in the polymer solutions to promote a gelation reaction in situ, and thus a gel-blood complex incorporating blood can be formed in a short time. As a result, it is possible to provide an excellent blood coagulation effect by taking blood into the gel, and a physical hemostatic effect by covering the bleeding site or the like with the gel.

Unlike in the prior art, the polymer material used in the present invention is not an animal-derived material and therefore can avoid a risk of infection or the like. Furthermore, there is an advantage that the present invention can be applied to a tissue having a complicated structure because after mixing two polymer solutions, the resulting mixture can maintain the liquid state for a certain period of time. In addition, there is also an advantage that the present invention can be applied to a patient to whom an anticoagulant or the like is administered because unlike a conventional method, the present invention can provide excellent blood coagulability not depending on the coagulability of the blood itself.

The present invention can be also applied to vascular occlusion by applying the in-situ gel formation of the present invention to a blood vessel such as a vein or an artery. Furthermore, the gel formation mechanism can exhibit a coagulation effect not only on blood but also on a body fluid having a pH near neutral because the gel formation mechanism utilizes a pH change due to application to a biological environment.

### Brief Description of Drawings

Fig. 1 is a graph showing an influence of concentration and pH on gelation time.
Fig. 2 is a graph showing a change in gelation time depending on ionic strength.
Fig. 3 is a graph showing a change in pH depending on ionic strength.
Fig. 4 is a graph showing a relation between gelation time and pH.
Fig. 5 is a graph showing a change in the Young's modulus caused by mixing with cow milk.
Fig. 6 is a graph showing a time-course change in swelling degree in cow milk.
Fig. 7 is a graph showing the equilibrium swelling degree at each prepolymer concentration in water.
Fig. 8 is an image in which a gel is applied to a venous blood vessel of a rat thigh.
Fig. 9 is an image in which a gel is applied to an arterial blood vessel of a rat abdomen.

### Description of Embodiments

Hereinafter, embodiments of the present invention will be described. The scope of the present invention is not limited by these descriptions and can be appropriately changed, and embodiments other than the following examples can be implemented without impairing the gist of the present invention.

### 1. Polymer Material Kit of the Present Invention

The polymer material kit of the present invention includes a polymer solution A containing a first polymer and a second polymer solution B containing a second polymer, and each polymer solution contains, under a specific concentration condition, a hydrophilic polymer capable of forming a hydrogel by intermolecular crosslinking and has a specific pH condition and specific ionic strength.

Under such solution conditions, the gelation reaction does not proceed in a short time even if these polymer solutions A and B are mixed as they are, but if the two solutions are mixed in a solution having a pH near neutral such as blood, gelation is promoted and caused in situ in a relatively short time and thus a hydrogel incorporating the blood into the gel (also referred to as a "gel-blood complex" or a "gel-body fluid complex") can be formed. It can be said that the polymer material kit of the present invention is a novel technique that has been unavailable heretofore because by forming such a gel-blood complex in situ, it is possible to provide both the blood coagulation effect by taking the blood itself into the gel and the physical hemostatic effect by covering the bleeding site or the like with the gel.

Hereinafter, polymer materials and solution conditions used in the polymer material kit of the present invention will be described in detail.

### (1-1) Polymer Materials

The first polymer and the second polymer used in the polymer solutions A and B of the present invention are hydrophilic polymers having a polyalkylene glycol skeleton or a polyvinyl skeleton that are capable of forming a hydrogel when crosslinked with each other. As the hydrophilic polymers, those known in the art can be used as long as a hydrogel can be formed by a gelation reaction (crosslinking reaction or the like) in an aqueous solution, and more specifically, polymers are preferable that are capable of forming a network structure, particularly a three-dimensional network structure when the polymers are crosslinked with each other in the final gel.

Typical examples of the polymer having a polyethylene glycol skeleton include polymer species having a plurality of branches having a polyethylene glycol skeleton, and a two-, three-, or four-armed polyethylene glycol is particularly preferable. In particular, a gel including a four-armed polyethylene glycol skeleton is generally known as a Tetra-PEG gel, and a network structure is constructed by an AB cross-end coupling reaction between two four-armed polymers having an electrophilic functional group such as an active ester structure and a nucleophilic functional group such as an amino group at the terminal respectively (Matsunaga et al., Macromolecules, Vol. 42, No. 4, pp. 1344-1351, 2009). The Tetra-PEG gel can be easily prepared in situ by simple two-liquid mixing of the polymer solutions, and the gelation time can also be controlled by adjusting the pH and the ionic strength at the time of gel preparation. This gel contains PEG as a main component, and therefore is also excellent in biocompatibility.

Examples of the hydrophilic polymer having a polyvinyl skeleton include polyalkyl methacrylates such as polymethyl methacrylate, polyacrylates, polyvinyl alcohols, poly N-alkyl acrylamides, and polyacrylamide.

The first polymer and the second polymer have a weight average molecular weight (Mw) in the range of 1 × 10³ to 1 × 10⁵, preferably in the range of 0.5 × 10⁴ to 5 × 10⁴, and more preferably in the range of 1 × 10⁴ to 2 × 10⁴.

The first polymer and the second polymer are preferably a combination of a polymer having one or more nucleophilic functional groups in the side chain or at the terminal and a polymer having one or more electrophilic functional groups in the side chain or at the terminal. For example, the first polymer preferably has one or more nucleophilic functional groups in the side chain or at the terminal, and the second polymer preferably has one or more electrophilic functional groups in the side chain or at the terminal. The nucleophilic functional group and the electrophilic functional group are crosslinked to form a gel. Here, the total number of the nucleophilic functional groups and the electrophilic functional groups is preferably 5 or more. These functional groups are more preferably present at the terminal.

Examples of the nucleophilic functional group present in the first and the second polymers include a thiol group (-SH) and an amino group, and those skilled in the art can appropriately use a known nucleophilic functional group. The nucleophilic functional group is preferably a -SH group. The nucleophilic functional groups may be the same or different, but are preferably the same. If the functional groups are the same, the reactivity with an electrophilic functional group to be crosslinked becomes uniform and a gel having a uniform three-dimensional structure is easily obtained.

As the electrophilic functional group present in the first and the second polymers, an active ester group can be used. Examples of such an active ester group include a maleimidyl group, an N-hydroxy-succinimidyl (NHS) group, a sulfosuccinimidyl group, a phthalimidyl group, an imidazoyl group, an acryloyl group, a nitrophenyl group, and - CO₂PhNO₂ (Ph represents an o-, m-, or p-phenylene group), and those skilled in the art can appropriately use another known active ester group. The electrophilic functional group is preferably a maleimidyl group. The electrophilic functional groups may be the same or different, but are preferably the same. If the functional groups are the same, the reactivity with a nucleophilic functional group to be crosslinked becomes uniform and a gel having a uniform three-dimensional structure is easily obtained.

Preferred non-limiting specific examples of the polymer having a nucleophilic functional group at the terminal include a compound that has four branches having a polyethylene glycol skeleton and has a thiol group at the terminal and is represented by the following formula (I).

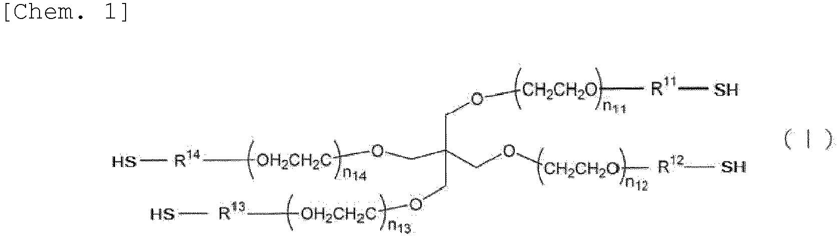

In the formula (I), R¹¹ to R¹⁴ are the same or different and each represent a C₁ to C₇ alkylene group, a C₂ to C₇ alkenylene group, -NH-R¹⁵-, -CO-R¹⁵-, -R¹⁶-O-R¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, or -R¹⁶-CO-NH-R¹⁷-, wherein R¹⁵ represents a C₁ to C₇ alkylene group, R¹⁶ represents a C₁ to C₃ alkylene group, and R¹⁷ represents a C₁ to C₅ alkylene group.
n₁₁ to n₁₄ may be the same or different. The closer the values of n₁₁ to n₁₄ are, the more uniform the three-dimensional structure can be, and the higher the strength becomes. Therefore, in order to obtain a gel having high strength, n₁₁ to n₁₄ are preferably the same. If the values of n₁₁ to n₁₄ are too high, the gel has low strength, and if the values of n₁₁ to n₁₄ are too low, the gel is less likely to be formed due to the steric hindrance of the compound. Therefore, the values of n₁₁ to n₁₄ are, for example, an integer value of 5 to 600, preferably 25 to 250, more preferably 50 to 120, and still more preferably 110 to 120.

In the formula (I), R¹¹ to R¹⁴ are linker moieties each connecting a functional group and a core moiety. R¹¹ to R¹⁴ may be the same or different, but are preferably the same in order to produce a gel having a uniform three-dimensional structure and high strength. R¹¹ to R¹⁴ each represent a C₁ to C₇ alkylene group, a C₂ to C₇ alkenylene group, -NH-R¹⁵-, -CO-R¹⁵-, -R¹⁶-O-R¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, or -R¹⁶-CO-NH-R¹⁷-. Here, R¹⁵ represents a C₁ to C₇ alkylene group. R¹⁶ represents a C₁ to C₃ alkylene group. R¹⁷ represents a C₁ to C₅ alkylene group.

Here, the term "C₁ to C₇ alkylene group" means an alkylene group that has 1 or more and 7 or less carbon atoms and may have a branch, and means a linear C₁ to C₇ alkylene group or a C₂ to C₇ alkylene group having one or more branches (having 2 or more and 7 or less carbon atoms including carbon atoms in the branches). Examples of the C₁ to C₇ alkylene group include a methylene group, an ethylene group, a propylene group, and a butylene group. Examples of the C₁ to C₇ alkylene group include -CH₂-, - (CH₂)₂-, -(CH₂)₃-, -CH(CH₃)-, -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, - (CH₂)₂-C(C₂H₅)₂-, and -(CH₂)₃C(CH₃)₂CH₂-.

The term "C₂ to C₇ alkenylene group" refers to a linear or branched alkenylene group having 2 to 7 carbon atoms and having 1 or more double bonds in the chain, and examples of the C₂ to C₇ alkenylene group include divalent groups having 1 or more double bonds formed by excluding 2 to 5 hydrogen atoms of adjacent carbon atoms from the alkylene groups.

Meanwhile, preferred non-limiting specific examples of the polymer having an electrophilic functional group at the terminal include a compound that has four branches having a polyethylene glycol skeleton and has an N-hydroxy-succinimidyl (NHS) group at the terminal and is represented by the following formula (II).

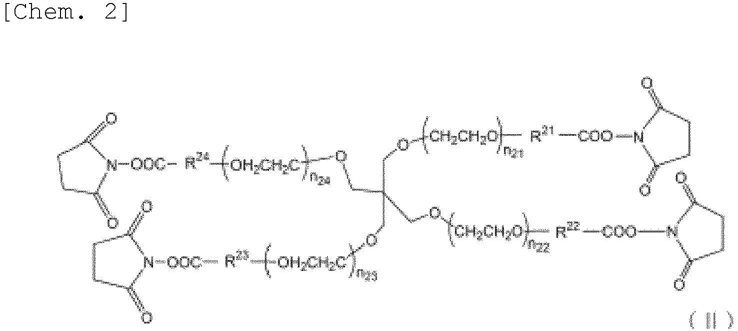

In the formula (II), n₂₁ to n₂₄ may be the same or different. The closer the values of n₂₁ to n₂₄ are, the more uniform three-dimensional structure and the higher strength the gel can have, and therefore the values are preferably close, and more preferably the same. If the values of n₂₁ to n₂₄ are too high, the gel has low strength, and if the values of n₂₁ to n₂₄ are too low, the gel is less likely to be formed due to the steric hindrance of the compound. Therefore, the values of n₂₁ to n₂₄ are, for example, an integer value of 5 to 600, preferably 25 to 250, more preferably 50 to 120, and still more preferably 110 to 120.

In the formula (II), R²¹ to R²⁴ are linker moieties each connecting a functional group and a core moiety. R²¹ to R²⁴ may be the same or different, but are preferably the same in order to produce a gel having a uniform three-dimensional structure and high strength. In the formula (II), R²¹ to R²⁴ are the same or different, and each represent a C₁ to C₇ alkylene group, a C₂ to C₇ alkenylene group, -NH-R²⁵-, -CO-R²⁵-, -R²⁶-O-R²⁷-, -R²⁶-NH-R²⁷-, -R²⁶-CO₂-R²⁷-, -R²⁶-CO₂-NH-R¹⁷-, -R²⁶-CO-R²⁷-, or -R²⁶-CO-NH-R²⁷-. Here, R²⁵ represents a C₁ to C₇ alkylene group. R²⁶ represents a C₁ to C₃ alkylene group. R²⁷ represents a C₁ to C₅ alkylene group.

In the present description, the alkylene group and the alkenylene group may have one or more arbitrary substituents. Examples of the substituent include, but are not limited to, alkoxy groups, halogen atoms (any of a fluorine atom, a chlorine atom, a bromine atom, and an iodine atom), an amino group, mono- or disubstituted amino groups, substituted silyl groups, acyl groups, and aryl groups. In the case of an alkyl group having two or more substituents, they may be the same or different. The same applies to alkyl moieties in other substituents having alkyl moieties (such as alkyloxy groups and aralkyl groups) .

In the present description, in a case where a certain functional group is defined as a functional group that may have a substituent, the kind of the substituent, the substitution position, and the number of substituents are not particularly limited, and in the case of a functional group having two or more substituents, they may be the same or different. Examples of the substituent include, but are not limited to, alkyl groups, alkoxy groups, a hydroxyl group, a carboxyl group, halogen atoms, a sulfo group, an amino group, alkoxycarbonyl groups, and an oxo group. These substituents may further have a substituent.

As another aspect, one of the first polymer or the second polymer can be replaced with a low molecular weight compound. In this case, the low molecular weight compound has one or more nucleophilic functional groups or electrophilic functional groups in the molecule. Thus, for example, a low molecular weight compound having a nucleophilic functional group in the molecule is used in place of the first polymer and reacted with the second polymer having one or more electrophilic functional groups in the side chain or at the terminal, and as a result, the second polymer can gel. Examples of the "low molecular weight compound having a nucleophilic functional group in the molecule" include compounds having a thiol group in the molecule, and for example, dithiothreitol can be used.

### (1-2) Conditions of Polymer Solutions

The polymer solution A and the polymer solution B included in the polymer material kit of the present invention satisfy the following conditions of the polymer concentration, the pH, the ionic strength, and the like.

The concentration of the first polymer in the polymer solution A and the concentration of the second polymer in the polymer solution B are each in the range of 10 to 300 g/L, preferably in the range of 30 to 200 g/L, and more preferably in the range of 50 to 150 g/L. By adjusting the polymer concentrations, the gelation time can be set within a desired range. The concentrations of the first and the second polymers may be the same or different as long as the above-described range is satisfied, but are preferably the same.

The polymer solutions A and B are adjusted so that a mixed solution obtained by mixing these solutions is in an acidic region (pH: less than 7), and are preferably adjusted so that the pH is in the range of 3 to less than 7, and more preferably in the range of 3.2 to 5.0. Any one of the polymer solutions A and B preferably has a pH in the range of 3 to less than 7, and preferably in the range of 3.2 to 5.0. However, as long as the mixed solution of the polymer solutions A and B satisfies the above-described acidic pH range, the other solution can have a pH of more than 8. In a typical aspect, both the polymer solutions A and B preferably have a pH in these ranges. With such a pH in an acidic side, no gelation reaction is caused (preferably, a gelation reaction does not proceed) in a short time by only mixing the polymer solutions A and B as they are, but in a case where the two solutions are mixed in an environment where blood or the like is present and the pH is near neutral (including a case where the mixed solution is applied to an environment having a pH near neutral), the pH of the polymer solution increases, and a gelation reaction can proceed. Thus, a gel can be formed in situ in a relatively short time in an environment where blood or the like is present and the pH is near neutral. The pH of the polymer solution A and the pH of the polymer solution B may be the same or different as long as the above-described range is satisfied, but are preferably the same.

The pH of the polymer solutions A and B can be set using a pH buffer known in the art. For example, a citrate-phosphate buffer (CPB) is used and the mixing ratio of citric acid and disodium hydrogen phosphate is changed, and thus the pH can be adjusted to the above-described range.

The polymer solutions A and B are adjusted so that a mixed solution obtained by mixing the polymer solutions A and B has an ionic strength in the range of 10 to 100 mM, and preferably in the range of 10 to 40 mM. By adjusting the ionic strength, the gelation time can be set within a desired range. The ionic strength of the polymer solution A and the ionic strength of the polymer solution B may be the same or different as long as the above-described range is satisfied, but are preferably the same.

If the conditions of the polymer concentration, the pH, and the ionic strength are set within the above-described range in each of the polymer solutions A and B, a hydrogel in which the first polymer and the second polymer are crosslinked with each other can be formed in situ by mixing these solutions in an environment where a liquid is present that has a pH of 6.5 to 8.0, which corresponds to the pH of a body fluid such as blood.

The gelation time at this time is preferably in the range of 1 to 30 seconds, and more preferably in the range of 1 to 10 seconds. Again, the gelation time can be adjusted mainly by appropriately setting the polymer concentration, the pH, and the ionic strength in the polymer solution. Here, the term "gelation time" refers to a time required until the storage elastic modulus G' and the loss elastic modulus G" satisfy G' = G".

The solvents in the polymer solutions A and B are water, but in some cases, may be a mixed solvent containing an alcohol such as ethanol or another organic solvent. The polymer solutions A and B are preferably an aqueous solution in which water is used as a single solvent.

The volumes of the polymer solutions A and B in the polymer material kit of the present invention can be appropriately adjusted according to, for example, the area and the structure complexity of the bleeding site, the blood vessel, or the like to which the polymer solutions A and B are applied, and are typically in the range of 0.1 to 20 ml, and preferably 1 to 10 ml.

### (1-3) Hydrogel

As described above, the first polymer and the second polymer can be crosslinked with each other to form a hydrogel. In the present description, the term "gel" generally refers to a dispersion system of a polymer having no fluidity, and refers to a state in which storage elastic modulus G' and the loss elastic modulus G" have a relation of G' ≥ G". The term "hydrogel" refers to a gel containing water.

The hydrogel formed using the first polymer and the second polymer preferably has an equilibrium swelling degree in the range of 0.9 to 3.5, and more preferably in the range of 0.9 to 2.5. As a result, after the hydrogel is formed in a bleeding site, a blood vessel, or the like, the gel does not excessively expand, and when the gel remains in the affected part for a certain period of time, an undesirable influence can be suppressed. Here, the term "equilibrium swelling degree" refers to a value of the swelling degree when a change in the swelling degree with time after gel formation reaches an equilibrium state. The swelling degree can be measured with a method commonly used in the art. As the swelling degree, a value measured at 25°C can be used.

The hydrogel formed using the first polymer and the second polymer preferably has a Young's modulus in the range of 0.1 × 10⁴ to 4 × 10⁴ Pa, and more preferably in the range of 0.5 × 10⁴ to 2 × 10⁴ Pa. As a result, the hydrogel formed in a bleeding site, a blood vessel, or the like can have an appropriate strength to remain in the affected part for a certain period of time.

### 2. Hemostatic Agent and the Like, and Hemostatic Method and the Like of the Present Invention

In another aspect, the present invention also relates to a hemostatic agent, a vascular occlusion agent, a tissue covering agent, or a body fluid coagulant including the polymer material kit.

As described above, a gel-blood complex incorporating blood can be formed using the polymer material kit of the present invention by applying the polymer solutions A and B to an environment where blood is present such as a bleeding site or the interior of a blood vessel to form a gel in situ. As a result, it is possible to provide an excellent blood coagulation effect by taking blood into the gel, and a physical hemostatic effect by covering the bleeding site or the like with the gel. Applying such in-situ gel formation to a blood vessel such as a vein or an artery enables use for vascular occlusion, and enables use for coagulation of not only blood but also a body fluid having a pH near neutral.

Here, the term "tissue" showing a target of the present invention can broadly mean a living tissue and a living organ where a liquid having a pH near neutral is present, and examples of the tissue include organs, nerves, muscles, and a part thereof. However, even a site having a surface that is generally considered to be acidic originally can be included in examples of the tissue as a target of the present invention when the pH is temporarily or permanently modified to a pH near neutral by a prior treatment. Examples of such a site include, but are not limited to, a gastric mucosa.

From another viewpoint, the present invention also relates to a method for producing such a hemostatic agent, vascular occlusion agent, tissue covering agent, or body fluid coagulant. The method is characterized by including a step of applying a polymer solution A containing a first polymer and a second polymer solution B containing a second polymer to an environment where a liquid having a pH near neutral, that is, a pH of 6.5 to 8.0 is present. In addition, the kinds of the first and second polymers and the conditions of the polymer solutions A and B are as described above.

Here, the "environment where a liquid having a pH of 6.5 to 8.0 is present" is preferably a place where blood or a body fluid is present, and can be, for example, a blood vessel such as an artery or a vein, or a tissue where blood or a body fluid is present. The range of the pH can be preferably 6.5 to 7.5.

The step of "applying" a mixed solution of the polymer solution A and the second polymer solution B to an environment where a liquid having a pH of 6.5 to 8.0 is present is typically exemplified by mixing the polymer solution A and the second polymer solution B in such a pH environment. Examples of the step include directly dripping or spraying the polymer solutions A and B in order on an affected part where blood is present. In some cases, the polymer solutions A and B may be dripped or sprayed simultaneously. As described above, the polymer solutions A and B are set to solution conditions under which no gelation reaction is caused (preferably, a gelation reaction does not proceed) in a short time by only mixing the polymer solutions A and B as they are, and therefore it is also possible to mix the polymer solutions A and B in advance to obtain one solution and then apply the solution to an environment where a liquid having a pH of 6.5 to 8.0 is present.

Another aspect of the step of "applying" can be exemplified by dripping the polymer solution A and the polymer solution B on a carrier and then bringing the carrier into contact with an environment where a liquid having a pH of 6.5 to 8.0 is present. This case includes once holding the polymer solutions in the carrier and then covering or protecting an affected part where blood is present (such as an affected part after suture) with the carrier. The carrier is not particularly limited as long as it includes a material capable of holding the polymer solutions, and examples of the carrier include cloth members such as gauze and members having absorbability such as sponge. Further modification example is also possible in which the carrier in a state of holding any one of the polymer solutions A and B is brought into contact with an affected part or the like and then the other polymer solution is dripped or sprayed on the carrier.

In a method of mixing the polymer solutions A and B by dripping, for example, a two-liquid mixing syringe as disclosed in WO 2007/083522 A can be used. The temperatures of the two liquids at the time of mixing is not particularly limited as long as the precursor units are dissolved and the liquids have fluidity. For example, the temperatures of the two liquids may be different, but are preferably the same because two liquids having the same temperature are easily mixed.

In a method of spraying the polymer solution A and/or the polymer solution B, a sprayer storing the solution can be used. As the sprayer, a known sprayer in the art can be appropriately used, and a medical sprayer is preferable. Therefore, such a sprayer can be used as a container in the kit of the present invention, and in this case, one aspect of the present invention can be a medical device and preferably a sprayer in which the polymer solutions A and B are stored.

It can also be said that performing the method for producing of the present invention corresponds to a hemostatic method, a vascular occlusion method, a tissue covering method, and a body fluid coagulation method in which the polymer material kit is used. Note that a tissue, a blood vessel, or the like that can be a target is not necessarily in a living body, and this method includes application to a tissue or the like taken out from the body by surgery or the like.

### Examples

Hereinafter, the present invention will be described in more detail with reference to Examples, but the present invention is not limited thereto.

### 1. Preparation of Polymer Solutions

As raw material polymers, tetrathiol-polyethylene glycol (Tetra-PEG-SH) having a -SH group at the terminal and tetramaleimidyl-polyethylene glycol (Tetra-PEG-MA) having a maleimidyl group at the terminal were used. As these raw material polymers, raw material polymers commercially available from NOF CORPORATION were used. Both the raw material polymers had a weight average molecular weight (Mw) of 20000.

As a buffer for polymer solutions, a citrate-phosphate buffer (CPB) was used. The mixing ratio of citric acid and disodium hydrogen phosphate was changed to adjust the pH. The molar concentrations (referred to as ionic strength) of citric acid and disodium hydrogen phosphate were changed to adjust the buffer capacity.

In the case of preparing a CPB having a pH of 3.8 and an ionic strength of 200 mM, each polymer solution was prepared with the following procedure.
1. A 200 mM aqueous citric acid solution and a 200 mM aqueous disodium hydrogen phosphate solution are prepared.
2. The aqueous citric acid solution and the aqueous disodium hydrogen phosphate solution are mixed at a ratio of aqueous citric acid solution : aqueous disodium hydrogen phosphate solution = 32.3 : 35.4.

In the case of a pH of 5.8, the solutions are mixed at a ratio of 19.7 : 60.6, and in the case of a pH of 3.0, the solutions are mixed at a ratio of 39.8 : 20.4.

The pH was finely adjusted by mixing these CPBs at a certain ratio. Each pH was measured using a pH meter (manufactured by HORIBA, Ltd.).

### 2. Gelation Experiment

The gelation behavior of the Tetra-PEG polymer was evaluated using cow milk having a pH equivalent to that of blood (pH = 7.4).

Two kinds of prepolymer solutions were each prepared in an amount of 1 mL, and the time from mixing of the solutions to gelation of the resulting mixture was measured, and thus the gelation time was examined. Here, the gelation time was simply defined as the time until the solution did not fall from the inverted container containing the solution. The concentration of the polymer and the pH and the ionic strength of the buffer were varied.

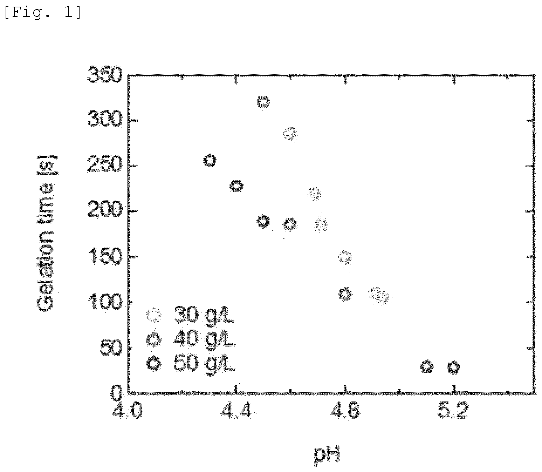

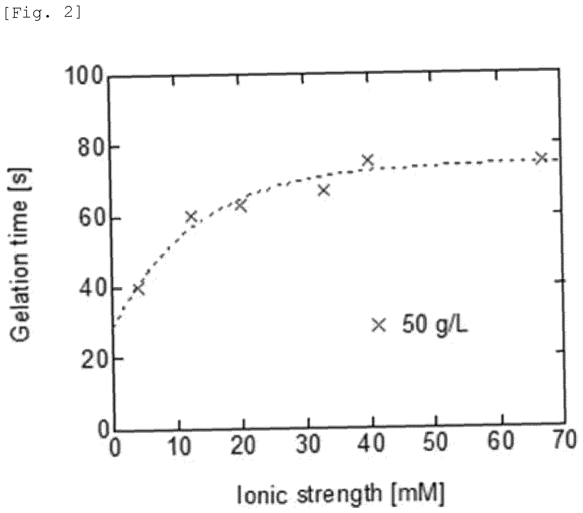

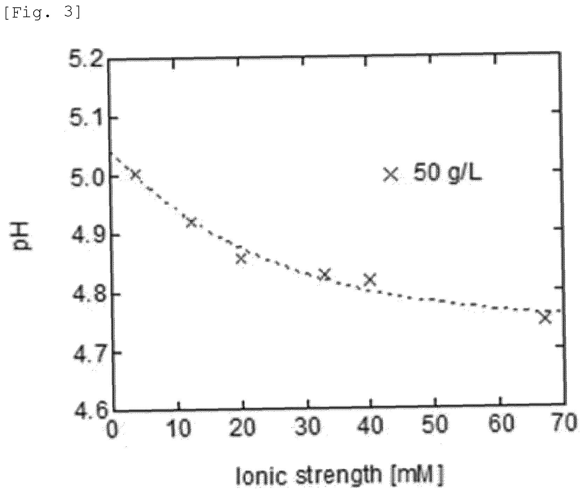

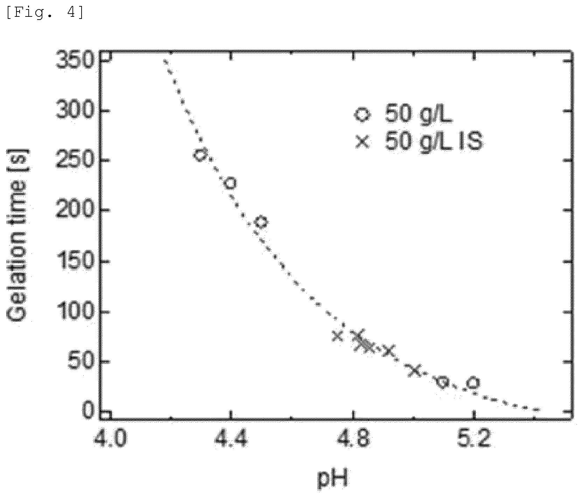

Fig. 1 shows the results of the dependence of the polymer concentration and the pH on the gelation time, and Fig. 2 shows the results of the dependence of the ionic strength on the gelation time. It has been found that the gelation time depends on the concentration of the prepolymer and the pH of the buffer. The gelation time also changes when the ionic strength of the buffer (which is an index of the strength of the ability to maintain the pH) is changed, but this is simply because the pH cannot be maintained as the ionic strength decreases. In fact, when the relation between the pH and the gelation time obtained from Figs. 2 and 3 is plotted on the graph of the relation between the pH and the gelation time at the same concentration, the same relation is obtained (Fig. 4).

Next, cow milk was subjected to a gelation test. First, a prepolymer solution was prepared for the present cow milk gelation test (prepolymer concentration: 150 g/L, solvent: pH 3.4, 200 mM). This prepolymer solution was added to cow milk (cow milk 10 : prepolymer solution 1; volume ratio), and the fluidity of the cow milk was evaluated macroscopically. The gelation time at this time was 3 minutes. In order to further adjust the gelation time, another prepolymer solution was prepared (prepolymer concentration: 200 g/L, solvent: pH 3.4, 40 mM). When the above test was performed using this prepolymer solution, the gelation time was shortened to 10 seconds.

Rat whole blood was subjected to a gelation test. First, a prepolymer solution was prepared for the present gelation test (prepolymer concentration: 200 g/L, solvent: pH 3.4, 40 mM). Whole blood collected from a rat was heparinized. The heparinized whole blood was divided into two parts, and to one part, the previously prepared prepolymer solution was added (whole blood 10 : prepolymer solution 1; volume ratio). To the other part, no prepolymer solution was added. At this time, gelation was observed only in the group to which the prepolymer solution was added.

The Tetra-PEG-SH was replaced with dithiothreitol, which has two SH groups (molecular weight: 154.253 g/mol), and a gelation test was performed. First, a prepolymer solution of Tetra-PEG-MA was prepared (prepolymer concentration: 200 g/L, solvent: pH 3.4, 40 mM). A dithiothreitol solution was newly prepared so that the terminal MA group of the prepolymer solution and the SH group of dithiothreitol were at the same molar concentration. When these two liquids having the same volume were mixed, the fluidity was lost, and gelation was confirmed.

The Tetra-PEG-SH was replaced with Linear-PEG-SH having two branches and having an SH group at the terminal, and a gelation test was performed. First, a prepolymer solution of Tetra-PEG-MA was prepared (prepolymer concentration: 200 g/L, solvent: pH 3.4, 40 mM). A Linear-PEG-SH solution was newly prepared so that the terminal MA group of the prepolymer solution and the SH group of Linear-PEG-SH were at the same molar concentration. When these two liquids having the same volume were mixed, the fluidity was lost, and gelation was confirmed.

The Tetra-PEG-MA was replaced with Linear-PEG-MA having two branches and having an MA group at the terminal, and a gelation test was performed. First, a prepolymer solution of Tetra-PEG-SH was prepared (prepolymer concentration: 200 g/L, solvent: pH 3.4, 40 mM). A Linear-PEG-MA solution was newly prepared so that the terminal SH group of the prepolymer solution and the MA group of Linear-PEG-MA were at the same molar concentration. When these two liquids having the same volume were mixed, the fluidity was lost, and gelation was confirmed.

### 3. Measurement of Young's Modulus

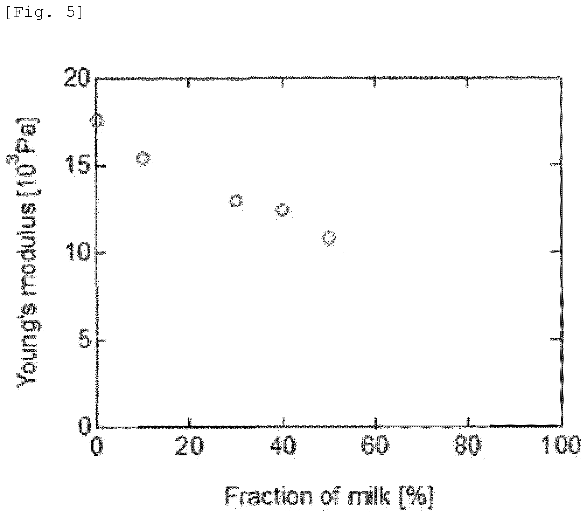

The prepolymer solution after mixing the two liquids is injected into the body and then mixed with blood, and thus gels. The change in the hardness (Young's modulus) depending on the content of blood at this time was examined. A prepolymer solution was prepared with the above-described procedure (prepolymer concentration: 50 g/L, solvent: pH 3.8, 200 mM). Two kinds of prepolymers were mixed, then cow milk was further mixed at various ratios, and after gelation and completion of the reaction, the Young's modulus was determined in a compression test (Fig. 5). For example, when cow milk having the same volume as the prepolymer solution is added to the prepolymer solution, the fraction of milk is expressed as 50%. The larger the volume of the added cow milk was, the lower the final Young's modulus was. This is because the addition of cow milk caused dilution of the prepolymer solution and reduction in the crosslinking point density of the gel.

### 4. Measurement of Equilibrium Swelling Degree

### Experimental procedure

Solvent: CPB (pH 3.0, 20 mM)
Prepolymer concentration: 60, 120 g/L (6, 12 wt%)

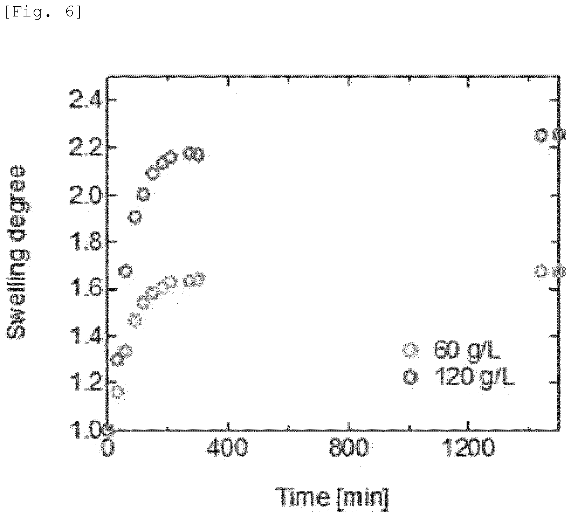

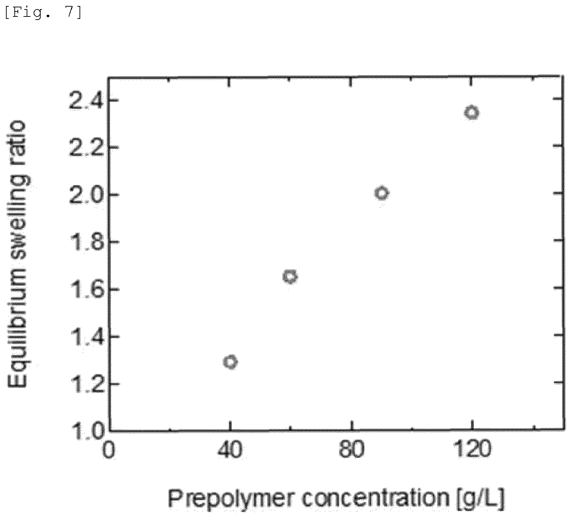

The gel preparation procedure is the same as described above. The two liquids were mixed, then after gelation and completion of the reaction, the mixture was put into cow milk, and the degree of swelling was examined. The gels at both concentrations reached an equilibrium swelling state in about 4 hours (Fig. 6). The equilibrium swelling degree was 1.7 at 60 g/L and 2.3 at 120 g/L, and these values were the same as those in the case of swelling in water (Fig. 7), suggesting that the results of the swelling experiment in water may be used.

### 5. Application to Rat Blood Vessel

### Experimental procedure

Solvent: CPB (pH 4.6, 20 mM) (mixed at CPB (pH 3.8, 20 mM) : CP (pH 5.8, 20 mM) = 1 : 2)
Prepolymer concentration: 50 g/L (5 wt%)

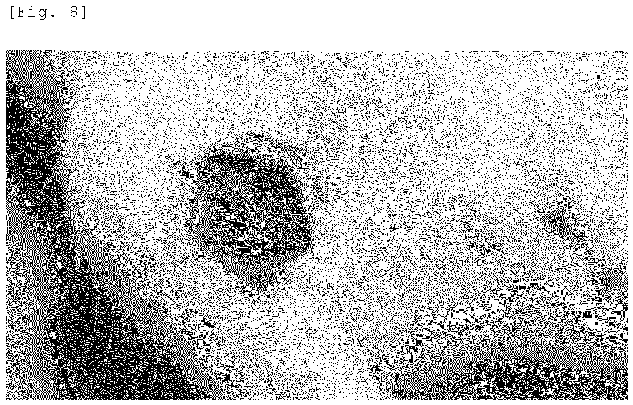

The gel preparation procedure is the same as described above. The two liquids were mixed, and then about 100 µL of the mixture was injected into a venous blood vessel of a rat thigh within about 3 minutes because the gelation time of the mixture in a state of being left to stand was about 3 minutes (this gelation time can be changed depending on the pH). At this time, the upstream part of the blood vessel was compressed to stop the blood flow, and thus the gel was prevented from flowing immediately after the injection. The ionic strength of the buffer was as low as 20 mM, and therefore the pH was easily increased when the solution was mixed with blood after the injection, and gelation occurred immediately. The state of compressing the upstream part of the blood vessel was maintained for about 30 seconds, then the hand was released, and it was confirmed that the gel was solidified and not flowing. After a lapse of a certain period of time, the affected part was opened again, and it was confirmed that the gel did not flow out. Fig. 8 shows the image at this time. It was confirmed that the effect of vascular occlusion by the gel lasted for at least 2 to 3 weeks.

In the case of using the conditions of the solvent: CPB (pH 3.0, 20 mM) and the prepolymer concentration: 100 g/L (10 wt%), a gelation time of 10 minutes or more was required after mixing the two liquids in a state of being left to stand, but the ionic strength was so low that the pH increased after mixing with rat blood, and gelation occurred immediately.

### 6. Experimental Procedure for Application to Abdominal Aorta of Rat

Solvent: CPB (pH 3.4, 40 mM)
Prepolymer concentration: 200 g/L (20 wt%)

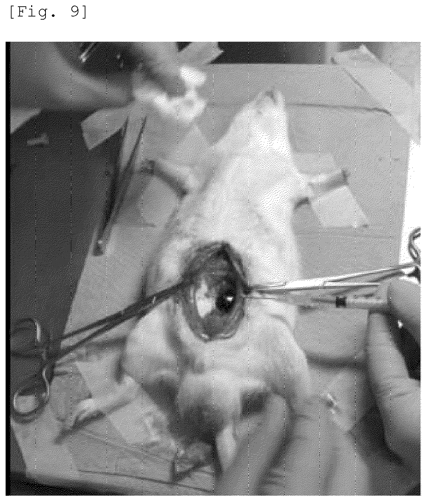

An abdominal aorta of a rat was punctured with an injection needle having an outer diameter of 0.2 mm to promote bleeding. A prepolymer solution after mixing the two liquids was added to the bleeding point (Fig. 9). In this state, pressure hemostasis was performed for 1 minute, and hemostasis was confirmed. At this time, as a comparative control group, pressure hemostasis was performed in the same manner for 1 minute without adding the prepolymer solution, and as a result, hemostasis was not observed.

## Claims

1. A polymer material kit comprising: a polymer solution A containing a first polymer; and a second polymer solution B containing a second polymer,
the first polymer and the second polymer being a hydrophilic polymer having a polyalkylene glycol skeleton or a polyvinyl skeleton, wherein a combination of the first polymer and the second polymer is capable of forming a hydrogel when the first polymer and the second polymer are crosslinked with each other,
the first polymer and the second polymer have a weight average molecular weight (Mw) in a range of 1 × 10³ to 1 × 10⁵,
the polymer solution A and the second polymer solution B have a concentration of the first polymer and a concentration of the second polymer in a range of 10 to 300 g/L, respectively, and
a mixed solution obtained by mixing the polymer solution A and the second polymer solution B has a pH in a range of 3 to less than 7 and an ionic strength in a range of 10 to 100 mM.

2. The polymer material kit according to claim 1, wherein the first polymer and the second polymer are a two-, three-, or four-armed polyethylene glycol.

3. The polymer material kit according to claim 1 or 2, wherein the first polymer has one or more nucleophilic functional groups in a side chain or at a terminal, and the second polymer has one or more electrophilic functional groups in a side chain or at a terminal.

4. The polymer material kit according to any one of claims 1 to 3, wherein the one or more nucleophilic functional groups are selected from the group consisting of a thiol group and an amino group, and the one or more electrophilic functional groups are selected from the group consisting of a maleimidyl group, an N-hydroxy-succinimidyl (NHS) group, a sulfosuccinimidyl group, a phthalimidyl group, an imidazoyl group, an acryloyl group, a nitrophenyl group, and -CO₂PhNO₂.

5. The polymer material kit according to any one of claims 1 to 4, wherein the polymer solution A and the second polymer solution B have a pH in a range of 3 to less than 7.

6. The polymer material kit according to any one of claims 1 to 5, wherein a hydrogel in which the first polymer and the second polymer are crosslinked with each other is formed when the polymer solution A and the second polymer solution B are mixed in an environment where a liquid having a pH of 6.5 to 8.0 is present.

7. The polymer material kit according to claim 6, wherein the hydrogel formed using the first polymer and the second polymer has a gelation time in a range of 1 to 30 seconds.

8. The polymer material kit according to claim 6 or 7, wherein the hydrogel formed using the first polymer and the second polymer has an equilibrium swelling degree in a range of 0.9 to 3.5.

9. The polymer material kit according to any one of claims 6 to 8, wherein the hydrogel formed using the first polymer and the second polymer has a Young's modulus in a range of 0.1 × 10⁴ to 4 × 10⁴ Pa.

10. The polymer material kit according to any one of claims 1 to 9, to be used for hemostasis, vascular occlusion, tissue covering, or body fluid coagulation.

11. A hemostatic agent comprising the polymer material kit according to any one of claims 1 to 10.

12. A vascular occlusion agent comprising the polymer material kit according to any one of claims 1 to 10.

13. A tissue covering agent comprising the polymer material kit according to any one of claims 1 to 10.

14. A body fluid coagulant comprising the polymer material kit according to any one of claims 1 to 10.

15. A method for producing a hemostatic agent, a vascular occlusion agent, a tissue covering agent, or a body fluid coagulant containing a hydrogel,
the method comprising a step of applying a mixed solution of a polymer solution A containing a first polymer and a second polymer solution B containing a second polymer to an environment where a liquid having a pH of 6.5 to 8.0 is present, wherein
the first polymer and the second polymer are a hydrophilic polymer having a polyalkylene glycol skeleton or a polyvinyl skeleton, a combination of the first polymer and the second polymer is capable of forming a hydrogel when the first polymer and the second polymer are crosslinked with each other,
the first polymer and the second polymer have a weight average molecular weight (Mw) in a range of 1 × 10³ to 1 × 10⁵,
the polymer solution A and the second polymer solution B have a concentration of the first polymer and a concentration of the second polymer in a range of 10 to 300 g/L, respectively, and
the mixed solution obtained by mixing the polymer solution A and the second polymer solution B has a pH of 3 to less than 7 and an ionic strength in a range of 10 to 100 mM.

16. The method according to claim 15, comprising mixing the polymer solution A and the second polymer solution B in an environment where a liquid having a pH of 6.5 to 8.0 is present.

17. The method according to claim 15, comprising dripping the polymer solution A and the second polymer solution B on a carrier and then bringing the carrier into contact with an environment where a liquid having a pH of 6.5 to 8.0 is present.

18. The method according to any one of claims 15 to 17, wherein the polymer solution A and the second polymer solution B have a pH in a range of 3 to less than 7.

19. A hemostatic method comprising using the polymer material kit according to any one of claims 1 to 10.

20. A vascular occlusion method comprising using the polymer material kit according to any one of claims 1 to 10.

21. A tissue covering method comprising using the polymer material kit according to any one of claims 1 to 10.

22. A body fluid coagulation method comprising using the polymer material kit according to any one of claims 1 to 10.

23. A medical device comprising the polymer material kit according to any one of claims 1 to 10.

24. The medical device according to claim 23, wherein at least one of the polymer solution A or the second polymer solution B is stored in a sprayer.
